(19) Europäisches Patentamt
European Patent Office
Office européen des brevets

(11) **EP 4 613 138 A1**

(12) **EUROPEAN PATENT APPLICATION**
published in accordance with Art. 153(4) EPC

(43) Date of publication:
**10.09.2025 Bulletin 2025/37**

(21) Application number: **22964472.9**

(22) Date of filing: **04.11.2022**

(51) International Patent Classification (IPC):
**A24F 40/65** (2020.01)     **A24F 40/53** (2020.01)
**A24F 40/57** (2020.01)

(52) Cooperative Patent Classification (CPC):
**A24F 40/53; A24F 40/57; A24F 40/65**

(86) International application number:
**PCT/JP2022/041181**

(87) International publication number:
**WO 2024/095455 (10.05.2024 Gazette 2024/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Japan Tobacco Inc.
Tokyo 105-6927 (JP)**

(72) Inventors:
• **FUJINAGA, Ikuo
  Tokyo 130-8603 (JP)**
• **TEZUKA, Hiroshi
  Tokyo 130-8603 (JP)**

(74) Representative: **Hoffmann Eitle
Patent- und Rechtsanwälte PartmbB
Arabellastraße 30
81925 München (DE)**

(54) **INFORMATION PROCESSING DEVICE, INHALATION SYSTEM, AND INFORMATION PRESENTATION METHOD**

(57)     A control unit (250) of a terminal device (200) capable of communicating with an inhalation device that delivers an aerosol which can be inhaled by a user performs the processes of: acquiring inhalation information representing an actual inhalation mode used for the inhalation device; generating, on the basis of the acquired inhalation information, comparison information in which the actual inhalation mode and a prescribed reference inhalation mode are compared; and presenting the generated comparison information to the user.

[FIG. 5]

FIG.5

EP 4 613 138 A1

## Description

[Technical Field]

The present disclosure relates to an information processing device, inhalation system, and information presentation method.

[BACKGROUND ART]

Conventionally, for example, inhalers which generate an aerosol having a flavor component and deliver the generated aerosol to a user in an inhalable manner are known. Such inhalers typically deliver the aerosol generated by heating a substrate, which comprises an aerosol source, with a heating unit (also referred to as a "heating element"), which is an electrically resistive or inductively heated heater. In recent years, research and development of temperature control of the heating unit in such inhalers and the function for communicating with external devices have been carried out (see, for example, PTL 1 and 2 below).

[CITATION LIST]

[PATENT LITERATURE]

[PTL 1] JP 2015-524260 A
[PTL 2] WO 2015/161402 A1

[SUMMARY OF INVENTION]

[TECHNICAL PROBLEM]

However, the history of research and development of inhalers is still young, and there is room for improvement in terms of providing a higher-quality experience for users.
The present disclosure provides an information processing device, inhalation system, and information presentation method that make it possible to provide a better quality experience to a user.

[SOLUTION TO PROBLEM]

One aspect of the present disclosure is an information processing device comprising a control unit that performs the processes of:

acquiring inhalation information representing an actual inhalation mode used for an inhalation device for delivering an aerosol which can be inhaled by a user;
generating, on the basis of the inhalation information, comparison information in which the actual inhalation mode and a prescribed reference inhalation mode are compared; and
presenting the comparison information to the user.

Another aspect of the present disclosure is

an inhalation system including the abovementioned information processing device and the inhalation device capable of communicating with the information processing device, wherein
the inhalation device transmits the inhalation information to the information processing device at a predetermined timing, and
the information processing device
is a terminal device that comprises a display unit capable of displaying information, and that is used by the user,
generating the comparison information on the basis of the inhalation information received from the inhalation device, and
presenting the comparison information to the user by causing the display unit to display the comparison information.

Another aspect of the present disclosure is

an information presentation method in which a computer performs the processes of:

acquiring inhalation information representing an actual inhalation mode used for an inhalation device for delivering an aerosol which can be inhaled by a user;

generating, on the basis of the inhalation information, comparison information in which the actual inhalation mode and a prescribed reference inhalation mode are compared; and

presenting the comparison information to the user.

[ADVANTAGEOUS EFFECTS OF INVENTION]

The present disclosure can provide an information processing device, inhalation system, and information presentation method that make it possible to provide a better quality experience to a user.

[BRIEF DESCRIPTION OF DRAWINGS]

Fig. 1 is a diagram showing an example of an inhalation system 10.

Fig. 2A is a diagram showing an example of an inhalation device 100A, which is a first configuration example of an inhalation device 100.

Fig. 2B is a diagram showing an example of an inhalation device 100B, which is a second configuration example of the inhalation device 100.

Fig. 3 is a diagram showing an example of a heating profile.

Fig. 4 is a diagram showing an example of inhalation information acquired by a control unit 116.

Fig. 5 is a diagram showing an example of a terminal device 200.

Fig. 6 is a flowchart showing an example of processes performed by a control unit 250.

Fig. 7 is a diagram showing an example of a preference information acquisition process.

Fig. 8A is a diagram showing an example of first reference inhalation information representing a first reference inhalation mode corresponding to option Op1.

Fig. 8B is a diagram showing an example of second reference inhalation information representing a second reference inhalation mode corresponding to option Op2.

Fig. 9 is a diagram showing one example of comparison information.

Fig. 10 is a diagram showing an example of inhalation assistance control.

Fig. 11 is a diagram showing an example of a heating profile Pr2 and a reference inhalation mode corresponding to the heating profile Pr2.

[DESCRIPTION OF EMBODIMENTS]

One embodiment of the information processing device, inhalation system, and information presentation method of the present disclosure will be described below in detail with reference to the drawings. The drawings shall be viewed in the orientation of the reference signs. It should be noted that the following embodiments do not limit the invention described in the claims and not all combinations of features described in the embodiments are essential to the invention. Furthermore, two or more of the plurality of features described in the embodiments may be optionally combined. It should be noted that, hereinafter, the same or similar elements may be given the same or similar reference signs, and the description thereof may be omitted or simplified as appropriate.

[1. Inhalation System]

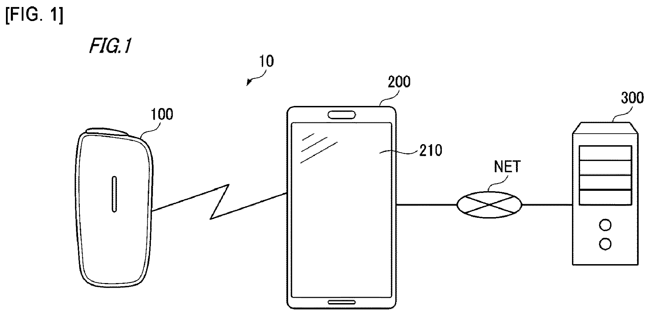

First, an example of an inhalation system of the present disclosure will be described. Fig. 1 is a diagram showing an example of an inhalation system 10. As shown in fig. 1, the inhalation system 10 includes an inhalation device 100 and a terminal device 200. In the inhalation system 10, the inhalation device 100 and the terminal device 200 are provided so as to be capable of communication with each other. For the communication between the inhalation device 100 and the terminal device 200, it is possible to use Wi-Fi (registered trademark), Bluetooth (registered trademark), Bluetooth Low Energy (BLE) (registered trademark), Near-Field Communication (NFC), Low Power Wide Area (LPWA), or the like. Furthermore, the inhalation device 100 and the terminal device 200 may be connected by a wired connection.

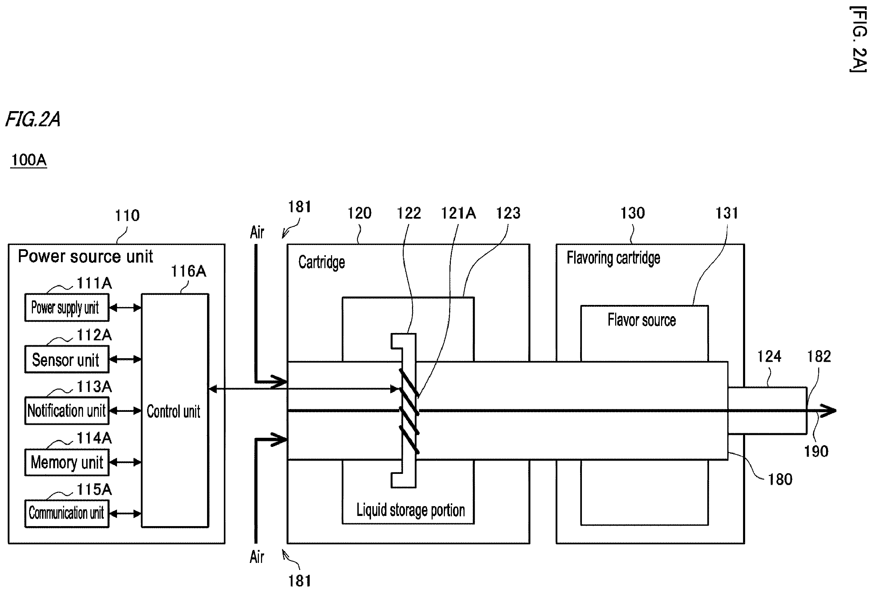

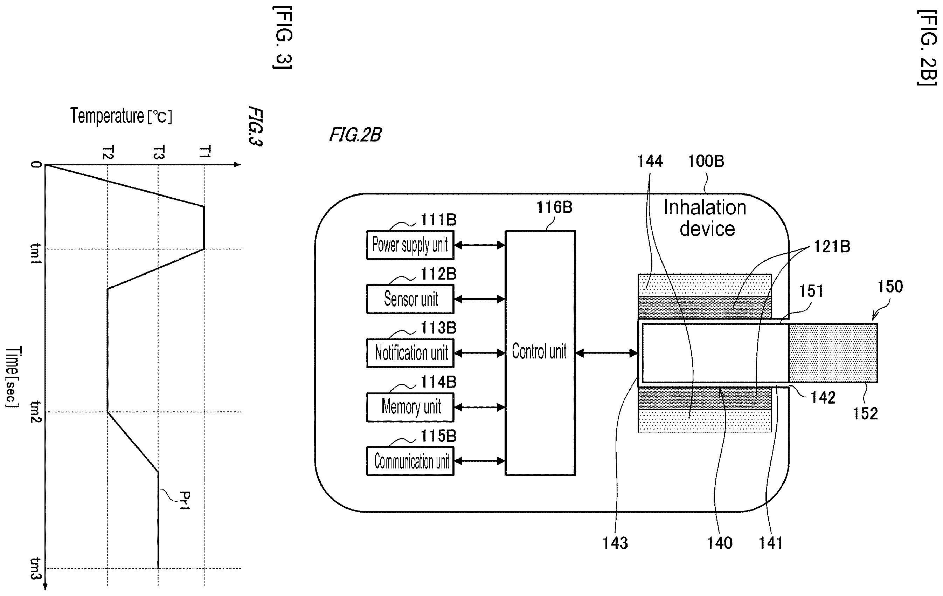

The inhalation device 100 is a device that generates a substance to be inhaled by a user and delivers the generated substance to the user in an inhalable manner. Hereinafter, the substance generated by the inhalation device 100 will be described as being an aerosol. Alternatively, the substance generated by the inhalation device 100 may be a gas. A specific configuration example of the inhalation device 100 will be described later using figs. 2A and 2B.

The terminal device 200 is an example of an information processing device of the present disclosure, which is a terminal device (computer) that comprises a display unit 210 capable of displaying information, and that is used by a user of the inhalation device 100. The inhalation device 100 and the terminal device 200 are linked to each other by the user, for example. The terminal device 200 can be a smartphone, a tablet terminal, a PC (Personal Computer), a wearable terminal (e.g. a smartwatch), or the like. Hereinafter, the terminal device 200 will be described as a smartphone.

The display unit 210 is composed of, for example, a liquid crystal display or an organic EL display, and, by displaying an image, presents information represented by the image to the user. Although details will be described later, the display unit 210 displays comparison information comparing the actual inhalation mode used for the inhalation device 100 and a prescribed reference inhalation mode. A specific configuration example of the terminal device 200 will be described later using fig. 3.

Furthermore, the terminal device 200 is provided so as to be capable of further communicating with a server 300, for example via a network NET. The network NET can be, for example, a mobile communication network, but is not limited thereto, and may be the Internet, a Wide Area Network (WAN), a LAN (Local Area Network) including Wi-Fi, or the like.

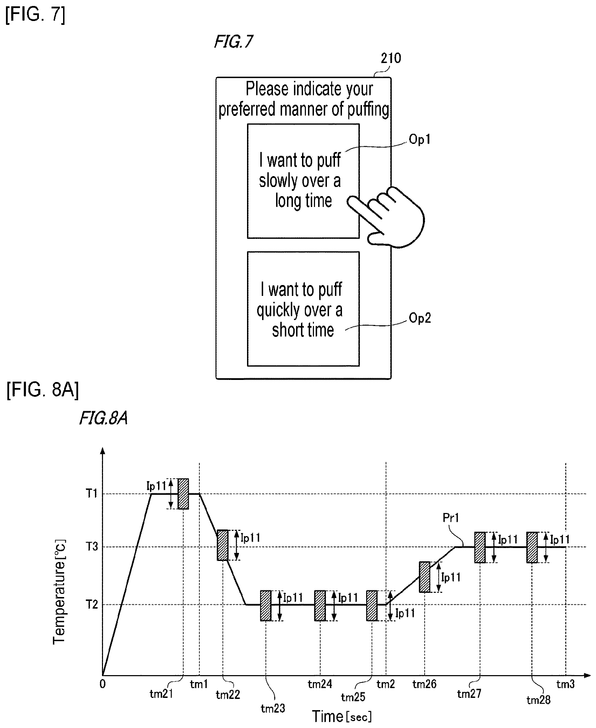

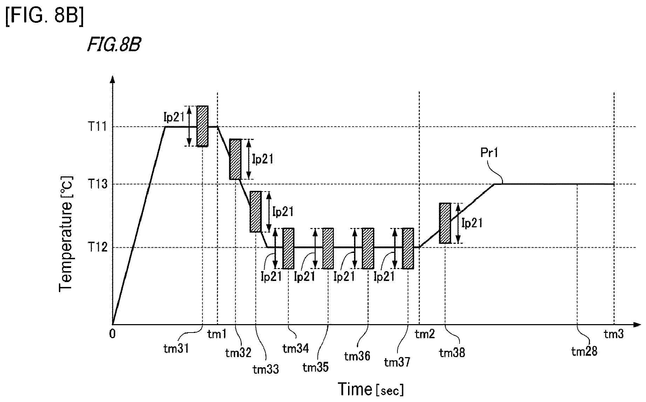

The server 300 is, for example, a server (computer) managed by the manufacturer of the inhalation device 100, and delivers predetermined information to the terminal device 200, etc. As an example, the server 300 delivers reference inhalation information representing the reference inhalation mode to the terminal device 200 in response to a delivery request from the terminal device 200. Specific examples of reference inhalation information will be described later using figs. 8A and 8B. Note that the server 300 may be a virtual server (cloud server) realized in a cloud computing service, or may be a physical server realized as a single device.

[2. Configuration Example of Inhalation Device]

<2-1. First Configuration Example of Inhalation Device>

A configuration example of the inhalation device 100 will be described next. Fig. 2A is a diagram showing an example of an inhalation device 100A, which is a first configuration example of the inhalation device 100. As shown in fig. 2A, the inhalation device 100A of the present example includes a power source unit 110, a cartridge 120, and a flavoring cartridge 130. The power source unit 110 comprises a power supply unit 111A, a sensor unit 112A, a notification unit 113A, a memory unit 114A, a communication unit 115A, and a control unit 116A. The cartridge 120 includes a heating unit 121A, a liquid guiding portion 122, and a liquid storage portion 123. The flavoring cartridge 130 includes a flavor source 131 and a mouthpiece 124. An air flow path 180 is formed in the cartridge 120 and the flavoring cartridge 130.

The power supply unit 111A stores electrical power. The power supply unit 111A then supplies the electric power to each component of the inhalation device 100A in accordance with control performed by the control unit 116A. The power supply unit 111A may be configured, for example, by a rechargeable battery such as a lithium ion secondary battery.

The sensor unit 112A acquires various types of information relating to the inhalation device 100A. The sensor unit 112A is configured by, for example, a pressure sensor such as a capacitor microphone, a flow rate sensor or a temperature sensor, etc., and acquires values associated with inhalation by a user.

As an example, the sensor unit 112A may include a pressure sensor (also referred to as a "puff sensor") that detects a change in pressure (also referred to hereinafter as "internal pressure") in the inhalation device 100 caused by inhalation by the user. As another example, the sensor unit 112A may include a flow sensor for detecting a flow rate caused by inhalation by the user (hereinafter simply referred to as "flow rate"). Furthermore, as another example, the sensor unit 112A may include a temperature sensor (also referred to as a "puff thermistor") that detects the temperature of the heating unit 121A or the temperature around the heating unit 121A.

Additionally, the sensor unit 112A can be configured by an input device, such as an operation button or switch, for accepting input of information from the user.

The notification unit 113A notifies the user of the information. The notification unit 113A can be configured by a light-emitting device which emits light, a display device which displays images, a sound output device which outputs sound, or a vibration device which vibrates, etc., for example.

The memory unit 114A stores various information (e.g. programs and data) for operation of the inhalation device 100A. The memory unit 114A can be configured by a non-volatile storage medium such as a flash memory, for example.

The communication unit 115A is a communication interface capable of performing communication in accordance with any wired or wireless communication standard. Standards such as Wi-Fi, Bluetooth, BLE, NFC, or LPWA, for example, may be adopted as the communication standard, The communication unit 115A communicates with a communication unit 240 of the terminal device 200 described below, for example. The communication unit 115A may also communicate with other devices (e.g., the server 300) other than the terminal device 200.

The control unit 116A functions as an arithmetic processing device and a control device, and controls overall operation within the inhalation device 100A in accordance with various programs stored in the memory unit 114A, etc. The control unit 116A is realized by a Central Processing Unit (CPU) or an electronic circuit such as a microprocessor, for example.

The liquid storage portion 123 stores an aerosol source. The aerosol source is atomized so as to generate an aerosol. The aerosol source is a polyhydric alcohol such as glycerol or propylene glycol, or a liquid such as water, for example. The aerosol source may include tobacco-derived or non-tobacco-derived flavor components. If the inhalation device 100A is a medical inhaler such as a nebulizer, the aerosol source may include a drug.

The liquid guiding portion 122 guides the aerosol source, which is the liquid stored in the liquid storage portion 123, from the liquid storage portion 123, and holds the aerosol source. The liquid guiding portion 122 is, for example, a wick formed by twisting a fibrous material such as glass fibers or a porous material such as a porous ceramic. In this case, the aerosol source stored in the liquid store portion 123 is guided by the capillary effect of the wick.

The heating unit 121A heats the aerosol source to atomize the aerosol source, thereby generating the aerosol. In the example shown in fig. 2A, the heating unit 121A is configured as a coil and wound around the liquid guiding portion 122. When the heating unit 121A generates heat, the aerosol source held in the liquid guiding portion 122 is then heated and atomized, generating the aerosol. The heating unit 121A generates heat when supplied with electricity from the power supply unit 111A. By way of example, electricity may be supplied when the sensor unit 112A detects that the user has started inhaling and/or that predetermined information has been input. The supply of electricity may then be stopped when the sensor unit 112A detects that the user has finished inhaling and/or that predetermined information has been input.

The flavor source 131 is a component for imparting a flavor component to the aerosol. The flavor source 131 may include tobacco-derived or non-tobacco-derived flavor components.

The air flow path 180 is a flow path for air to be inhaled by the user. The air flow path 180 has a tubular structure with an air inflow hole 181, which is an inlet for air into the air flow path 180, and an air outflow hole 182, which is an outlet for air from the air flow path 180. Partway through the air flow path 180, the liquid guiding portion 122 is disposed upstream (closer to the air inflow hole 181), and the flavor source 131 is disposed downstream (closer to the air outflow hole 182). Air flowing in through the air inflow hole 181 upon inhalation by the user is mixed with the aerosol generated by the heating unit 121A and transported through the flavor source 131 to the air outflow hole 182, as shown by the arrow 190. When the mixed fluid of aerosol and air passes through the flavor source 131, the flavor component contained in the flavor source 131 is applied to the aerosol.

The mouthpiece 124 is a member which is held in the user's mouth during inhalation. The air outflow hole 182 is disposed in the mouthpiece 124. The user holds the mouthpiece 124 in their mouth to make it possible to draw the mixed fluid of aerosol and air into the oral cavity.

A configuration example of the inhalation device 100A has been described above. The inhalation device 100A is, of course, not limited to the configuration described above, and may adopt various configurations, such as those illustrated below by way of example.

As an example, the inhalation device 100A need not include the flavoring cartridge 130. In this case, the cartridge 120 is provided with the mouthpiece 124.

As another example, the inhalation device 100A may include a plurality of types of aerosol sources. Other types of aerosol may be generated by a plurality of types of aerosol generated from the plurality of types of aerosol sources being mixed in the air flow path 180 to cause a chemical reaction.

Furthermore, the means for atomizing the aerosol source is not limited to heating provided by the heating unit 121A. For example, the means for atomizing the aerosol source may be vibration atomization or induction heating.

<2-2. Second Configuration Example of Inhalation Device>

Fig. 2B is a diagram showing an example of an inhalation device 100B, which is a second configuration example of the inhalation device 100. As illustrated in fig. 2B, the inhalation device 100B according to the present example comprises a power supply unit 111B, a sensor unit 112B, a notification unit 113B, a memory unit 114B, a communication unit 115B, a control unit 116B, heating units 121B, an accommodating portion 140, and a heat insulating portion 144.

The power supply unit 111B, sensor unit 112B, notification unit 113B, memory unit 114B, communication unit 115B, and control unit 116B are each substantially identical to the corresponding component included in the inhalation device 100A described above.

The accommodating portion 140 has an internal space 141, and holds a stick-type substrate 150 while accommodating a portion of the stick-type substrate 150 in the internal space 141. The accommodating portion 140 has an opening 142 allowing the internal space 141 to communicate with the outside, and accommodates the stick-type substrate 150 that has been inserted into the internal space 141 from the opening 142. For example, the accommodating portion 140 is a cylindrical body comprising the opening 142 and a bottom portion 143 serving as a bottom surface, and defines a columnar internal space 141. An air flow path for supplying air to the internal space 141 is connected to the accommodating portion 140. An air inflow hole, which is an inlet for air into the air flow path, is disposed in a side surface of the inhalation device 100, for example. An air outflow hole serving as an outlet for air from the air flow path to the internal space 141 is disposed in the bottom portion 143, for example.

The stick-type substrate 150 comprises a substrate portion 151 and a mouthpiece portion 152. The substrate portion 151 includes an aerosol source. The aerosol source comprises a tobacco-derived or non-tobacco-derived flavor component. If the inhalation device 100B is a medical inhaler such as a nebulizer, the aerosol source may include a drug. The aerosol source may be, for example, a liquid such as water and polyhydric alcohols such as glycerol and propylene glycol comprising the tobacco-derived or non-tobacco-derived flavor component, or else may be a solid comprising the tobacco-derived or non-tobacco-derived flavor component. In a state in which the stick-type substrate 150 is being held in the accommodating portion 140, at least a portion of the substrate portion 151 is accommodated in the internal space 141, and at least a portion of the mouthpiece portion 152 protrudes from the opening 142. Then, when the user holds the mouthpiece portion 152 protruding from the opening 142 in their mouth and inhales, air flows into the internal space 141 via the air flow path, which is not illustrated in the drawings, and reaches the inside of the user's mouth together with the aerosol generated from the substrate portion 151.

In the example illustrated in fig. 2B, the heating units 121B are configured in a film shape and are disposed so as to cover the outer periphery of the accommodating portion 140. Then, when the heating units 121B generate heat, the substrate portion 151 of the stick-type substrate 150 is heated from the outer periphery, generating the aerosol.

The heat insulating portion 144 prevents heat transfer from the heating units 121B to other components. For example, the heat insulating portion 144 is configured from a vacuum heat insulating material or an aerogel heat insulating material, or the like.

A configuration example of the inhalation device 100B has been described above. The inhalation device 100B is, of course, not limited to the configuration described above, and may adopt various configurations, such as those illustrated below by way of example.

As one example, the heating unit 121B may have a blade-like form and may be arranged so as to protrude into the internal space 141 from the bottom portion 143 of the accommodating portion 140. In this case, the blade-like heating unit 121B is inserted into the substrate portion 151 of the stick-type substrate 150 and heats the substrate portion 151 of the stick-type substrate 150 from the inside. As another example, the heating unit 121B may be arranged so as to cover the bottom portion 143 of the accommodating portion 140. Furthermore, the heating unit 121B may be configured by a combination of two or more from among a first heating unit covering the outer circumference of the accommodating portion 140, a blade-like second heating unit, and a third heating unit covering the bottom portion 143 of the accommodating portion 140.

As another example, the accommodating portion 140 may comprise an opening/closing mechanism such as a hinge for opening/closing part of a casing that forms the internal space 141. By opening/closing the casing, the accommodating portion 140 may then receive and grip the stick-type substrate 150 which has been inserted into the internal space 141. In this case, the heating units 121B may be provided on the clamping part of the accommodating portion 140, and may heat the stick-type substrate 150 while pressing the same.

Furthermore, the means for atomizing the aerosol source is not limited to heating provided by the heating units 121B. For example, the means for atomizing the aerosol source may be induction heating. In this case, the inhalation device 100B comprises at least an electromagnetic induction source such as a coil for generating a magnetic field, instead of the heating units 121B. A susceptor which generates heat by means of induction heating may be provided in the inhalation device 100B, or may be contained in the stick-type substrate 150.

The inhalation device 100B may further include the heating unit 121A, the liquid guiding portion 122, the liquid storage portion 123 and the air flow path 180 according to the first configuration example, and the air flow path 180 may supply air to the internal space 141. In this case, the mixed fluid of aerosol and air generated by the heating unit 121A flows into the internal space 141 and is further mixed with the aerosol generated by the heating units 121B to reach the oral cavity of the user.

[3. Operation Example of Inhalation Device]

<3-1. Generation of Aerosol>

An operation example of the inhalation device 100 (100A, 100B) will be described next. The control unit 116 (116A, 116B) of the inhalation device 100 can operate the inhalation device 100 on the basis of input from the user. As an example, the control unit 116 causes the inhalation device 100 to generate an aerosol in response to a request for aerosol generation from the user.

The request for aerosol generation can be, for example, an operation for instructing the start of heating (also referred hereinafter as a "heating start operation"). As an example, the heating start operation can be a press of a predetermined operation button (not shown) provided on the inhalation device 100. As another example, the heating start operation may be an inhalation operation on the inhalation device 100 while the inhalation device 100 is powered on. Furthermore, the request for aerosol generation is not limited to direct operation on the inhalation device 100, but may be, for example, the reception of predetermined information from another device that is able to communicate with the inhalation device 100, such as a smartphone. The control unit 116 can detect the request for aerosol generation based on information obtained by the sensor unit 112 (112A, 112B) or the communication unit 115 (115A, 115B), for example.

For example, if the inhalation device 100 is the inhalation device 100A shown in fig. 2A, the control unit 116A causes an aerosol to be generated by supplying predetermined power to the heating unit 121A upon detecting an inhalation operation on the inhalation device 100A on the basis of the detection result of a puff sensor. At this time, the power supplied to the heating unit 121A is predetermined by the manufacturer of the inhalation device 100A such that a suitable amount of aerosol including the appropriate amount of flavor component is generated. This makes it possible to provide a high-quality smoking experience to the user.

Furthermore, if the inhalation device 100 is the inhalation device 100B shown in fig. 2B, the control unit 116B causes an aerosol to be generated by controlling the temperature of the heating units 121B on the basis of a pre-prepared heating profile upon detecting a heating start operation (e.g., a press of a predetermined operation button). Here, the heating profile is information representing a heating mode of the stick-type substrate 150 (i.e., the aerosol source) by the heating units 121B, and, for example, the heating profile is information defining a time series transition of a target temperature, which is a target value of the temperature of the heating units 121B. It should be noted that the heating profile is pre-stored in the memory unit 114B, for example.

To elaborate on the temperature control of the heating units 121B that is based on the heating profile (also referred to simply as "heating control" hereinafter), the control unit 116B controls the temperature of the heating units 121B on the basis of the divergence between a target temperature corresponding to an elapsed time since the start of heating control, and the actual temperature of the heating unit 121B (also referred to as "actual temperature" hereinafter). More specifically, at this time, the control unit 116B controls the temperature of heating units 121B such that the time series transition of the actual temperature of the heating units 121B is similar to the time series transition of the target temperature defined in the heating profile.

The temperature control of the heating units 121B can be achieved by known feedback control, for example. The control unit 116B may cause power from the power supply unit 111B to be supplied to the heating units 121B in the form of pulses by pulse width modulation (PWM) or pulse frequency modulation (PFM), for example. In that case, the control unit 116B can perform temperature control of the heating units 121B by adjusting the duty ratio of the power pulse.

In the feedback control, the control unit 116B only needs to control the power supplied to the heating units 121B, e.g., said duty ratio, on the basis of the difference between the actual temperature and the target temperature, etc. Furthermore, the feedback control may be Proportional-Integral-Differential Controller (PID) control. Alternatively, the control unit 116B may perform simple ON-OFF control. For example, the control unit 116B may perform heating by the heating units 121B until the actual temperature reaches the target temperature, interrupt heating by the heating units 121B when the actual temperature reaches the target temperature, and resume heating by the heating units 121B when the actual temperature falls below the target temperature.

The temperature of the heating units 121B can be obtained (or in other words, quantified) by measuring or estimating the electrical resistance of heating resistive elements constituting the heating units 121B, for example. This is because the electrical resistance of the heating resistive element varies with temperature. The electrical resistance of the heating resistive element can be estimated (or namely, obtained) by measuring the amount of voltage drop at the heating resistive element, for example. The amount of voltage drop at the heating resistive element can be measured (or namely, obtained) by a voltage sensor measuring a potential difference applied to the heating resistive element. As another example, the temperature of the heating units 121B may be measured by a temperature sensor (puff thermistor) installed in the vicinity of the heating units 121B.

It should be noted that the heating profile is designed such that the flavor to be tasted by the user is optimal, when assuming that the user inhales the aerosol generated from the stick-type substrate 150 in a predetermined inhalation mode (e.g., the reference inhalation mode described later), for example. Thus, controlling the temperature of the heating units 121B on the basis of the heating profile can provide a high-quality smoking experience to the user inhaling in the predetermined inhalation mode.

Fig. 3 is a diagram showing an example of the heating profile. The vertical axis in fig. 3 represents the temperature [°C] of the heating units 121B. The horizontal axis in fig. 3 represents time [sec], and more specifically, the elapsed time since the start of heating control.

The control unit 116B performs heating control that is based on the heating profile Pr1 shown in fig. 3, for example. In the heating profile Pr1, the target temperature when the elapsed time since the start of heating control is greater than or equal to 0[sec] but less than tm1[sec] (where tm1>0. For example, 30[sec]) is T1[°C] (e.g., 290[°C]). Furthermore, the target temperature when the elapsed time since the start of heating control is greater than or equal to tm1[sec] but less than tm2[sec] (where tm2>tm1. For example, 180[sec]) is T2[°C] (where T2<T1. For example, 230[°C]). Additionally, the target temperature when the elapsed time since the start of heating control is tm2[sec] to tm3[sec] (where tm3>tm2. For example, 300[sec]) is T3[°C] (where T2<T3<T1. For example, 260[°C]).

Thus, according to the heating profile Pr1, as shown in fig. 3, the heating units 121B can be heated to T1[°C] in conjunction with the start of heating control, and then lowered in temperature to T2[°C], after which the heating unit can be heated again to T3[°C]. The heating control can then be ended when the elapsed time since the start of heating control is tm3[s].

Furthermore, there are also times when a predetermined number of inhalations (eight in the present embodiment) is performed before the elapse of tm3[sec] from the start of heating control. In this case, the control unit 116B ends the heating control upon the predetermined number of inhalations.

It should be noted that, hereinafter, unless otherwise specified, the inhalation device 100 of the present embodiment will be described as the inhalation device 100B shown in fig. 2B, wherein heating control that is based on the heating profile Pr1 is performed, but is not limited thereto.

<3-2. Acquisition and Transmission of Inhalation Information>

Furthermore, the control unit 116 obtains inhalation information representing the actual inhalation mode used for the inhalation device 100 on the basis of the information obtained by the sensor unit 112. Here, the actual inhalation mode includes, for example, the inhalation intensity and inhalation time pertaining to the inhalation performed on the inhalation device 100.

The inhalation intensity is an evaluation value representing the strength of inhalation intensity and can be the amount of change in internal pressure per unit time, for example. Alternatively, for example, the temperature drop amount of the heating units 121 per unit time, or the flow rate per unit time, can also be adopted as the inhalation intensity. Furthermore, the inhalation time is the time during which the inhalation was performed, and is a time specified by elapsed time since the start of heating control, for example.

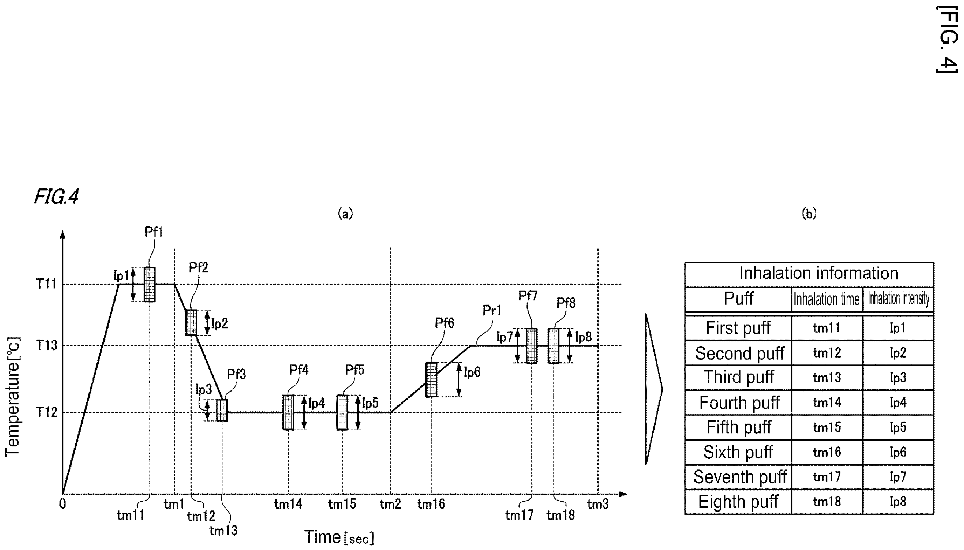

Fig. 4 is a diagram showing an example of inhalation information acquired by a control unit 116. Hereinafter, the inhalation on the inhalation device 100 is also referred to as a "puff".

As shown in (a) in fig. 4, a first puff Pf1, second puff Pf2, third puff Pf3, fourth puff Pf4, fifth puff Pf5, sixth puff Pf6, seventh puff Pf7 and eighth puff Pf8 are performed sequentially during heating control that is based on the heating profile Pr. Namely, the first puff Pf1 is the first puff taken during the heating control. Similarly hereinafter, the second puff Pf2 is the second puff, the third puff Pf3 is the third puff, the fourth puff Pf4 is the fourth puff, the fifth puff Pf5 is the fifth puff, the sixth puff Pf6 is the sixth puff, the seventh puff Pf7 is the seventh puff, and the eighth puff Pf8 is the eighth puff.

In (a) in fig. 4, the length in the vertical direction of the rectangle representing each puff from the first puff Pf1 to the eighth puff Pf8 is indicative of the inhalation intensity of each puff. For example, the inhalation intensity of the first puff Pf1 is Ip1. Furthermore, the inhalation time of the first puff Pf1 is when the elapsed time since the start of heating control is tm11 [sec].

When each puff from the first puff Pf1 to the eighth puff Pf8 shown in (a) in fig. 4 is taken, the control unit 116 obtains inhalation information associating the inhalation time and inhalation intensity of each puff from the first puff Pf1 (i.e., the first puff) to the eighth puff Pf8 (i.e., the eighth puff), as shown in (b) in fig. 4. The control unit 116 then transmits the inhalation information to the terminal device 200 at a predetermined timing.

In the present embodiment, at the end of the heating control, the control unit 116 transmits, to the terminal device 200, inhalation information associating the inhalation time and the inhalation intensity of each puff taken during the heating control. This allows the terminal device 200 to generate and present comparison information, which is described later, to the user each time heating control is performed (e.g., every 8 puffs).

The control unit 116 may also be configured to transmit inhalation information to the terminal device 200 each time a plurality of rounds of heating control is performed or each time a predetermined time has elapsed (e.g., every day). **In** this case, the control unit 116 may transmit inhalation information indicating the average value of the inhalation time of the nth puff in each of the plurality of rounds of heating control instead of the inhalation time of the nth (where n is a natural number of 1 or more) puff in a single round of heating control, and the average value of the inhalation intensity of the nth puff in each of the plurality of rounds of heating control instead of the inhalation intensity of the nth puff in a single round of heating control. **In** this way, the terminal device 200 can obtain inhalation information indicative of an averaged inhalation time and inhalation intensity (in other words, an averaged inhalation mode), and can present comparison information that is based on the averaged inhalation mode.

Furthermore, the inhalation information may further include other information. For example, the inhalation information may include information indicative of an inhalation period pertaining to inhalation performed on the inhalation device 100. Here, the inhalation period is an evaluation value representing the duration per inhalation (i.e., per puff), and can be the length of the period (time) during which the internal pressure that drops with inhalation was below or equal to a threshold value, for example. Alternatively, for example, the length of time during which the temperature of the heating unit 121, which decreases with inhalation, is less than or equal to a threshold value can be adopted as the inhalation period.

Furthermore, the inhalation information may include information indicative of an inhalation interval pertaining to inhalation performed on the inhalation device 100. Here, the inhalation interval is an evaluation value representing the length of a time interval (in other words, interval) between one puff taking place and the next puff taking place, and can be the length of time during which the inhalation intensity described above was less than or equal to a threshold value between each puff, for example.

[4. Configuration Example of Terminal Device]

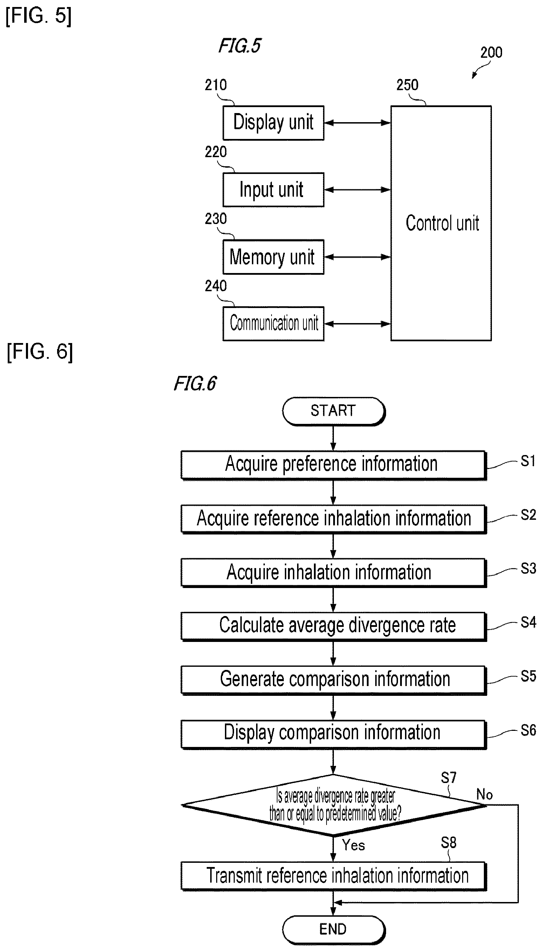

A configuration example of the terminal device 200 will be described next. Fig. 5 is a diagram showing an example of the terminal device 200. As shown in Fig. 5, the terminal device 200 includes a display unit 210, an input unit 220, a memory unit 230, a communication unit 240, and a control unit 250.

The display unit 210 presents various information (e.g., images or text) to the user by displaying the information on the basis of control by the control unit 250. **In** the present embodiment, the display unit 210 displays comparison information, which is described later.

The input unit 220 is configured by an input device such as a touch panel, keyboard, or mouse, and accepts input of information (operation input) from a user. **In** the present embodiment, the input unit 220 includes a touch panel integrally provided with the display as the display unit 210.

The memory unit 230 stores various information (e.g., programs and data) for operation of the terminal device 200. For example, the memory unit 230 can store inhalation information received by the terminal device 200 from the inhalation device 100, and reference inhalation information (described later) received by the terminal device 200 from the server 300. Note that the memory unit 230 is configured by a non-volatile storage medium such as a flash memory, for example.

The communication unit 240 is a communication interface capable of performing communication in accordance with any wired or wireless communication standard. Standards such as Wi-Fi, Bluetooth, BLE, NFC, or LPWA, for example, may be adopted as the communication standard, The communication unit 240 communicates with the communication unit 115 (115A, 115B) of the terminal device 100, for example. Furthermore, in the present embodiment, the communication unit 240 is also configured to be able to communicate with other devices (e.g. the server 300) via a network NET.

The control unit 250 functions as an arithmetic processing device and a control device, and controls overall operation within the terminal device 200 in accordance with various programs stored in the memory unit 230, etc. The control unit 250 is realized by a CPU or an electronic circuit such as a microprocessor, for example.

[5. Operation Example of Terminal Device]

An operation example of the terminal device 200 will be described next. The history of research and development of inhalers such as the inhalation device 100 is still young, and there is room for improvement in terms of providing a better quality experience for users. For example, some users wanted a more entertaining experience that went a step further from the experience of "inhaling an aerosol".

Accordingly, the control unit 250 of the terminal device 200 obtains inhalation information representing the actual inhalation mode used for the inhalation device 100, generates, on the basis of the acquired inhalation information, comparison information in which the actual inhalation mode and a prescribed reference inhalation mode are compared, and presents the generated comparison information to said user. This makes it possible for the comparison information to indicate, to the user, the degree of divergence of the actual inhalation mode from the reference inhalation mode, thereby enabling a new enjoyment to be provided to the user, such as seeking an inhalation mode closer to the reference inhalation mode with reference to this degree of divergence. Thus, it is possible to provide a more pleasurable and high-quality experience to the user and improve the merchantability of the inhalation device 100.

The reference inhalation mode can be an inhalation mode in which the flavor to be tasted when the user inhales the aerosol generated by the inhalation device 100 is optimal, for example. This allows a higher-quality smoking experience to be provided to a user who performed inhalation in an inhalation mode close to the reference inhalation mode.

It is also assumed, for example, that the desired flavor strength and the manner of inhalation differ from user to user. Thus, the reference inhalation mode may be different depending on the result of input by the user (e.g., operation input). In this way, it is possible to indicate to the user the degree of divergence of the actual inhalation mode from the reference inhalation mode reflecting the user's preferences.

The method for presenting the comparison information (presentation mode) is not particularly limited as long as the degree of divergence of the actual inhalation mode from the reference inhalation mode can be indicated to the user. For example, the comparison information can be presented by an image, sound, vibration by a vibrating device, light emission from a light emitting element (e.g., LED: light emitting diode), etc.

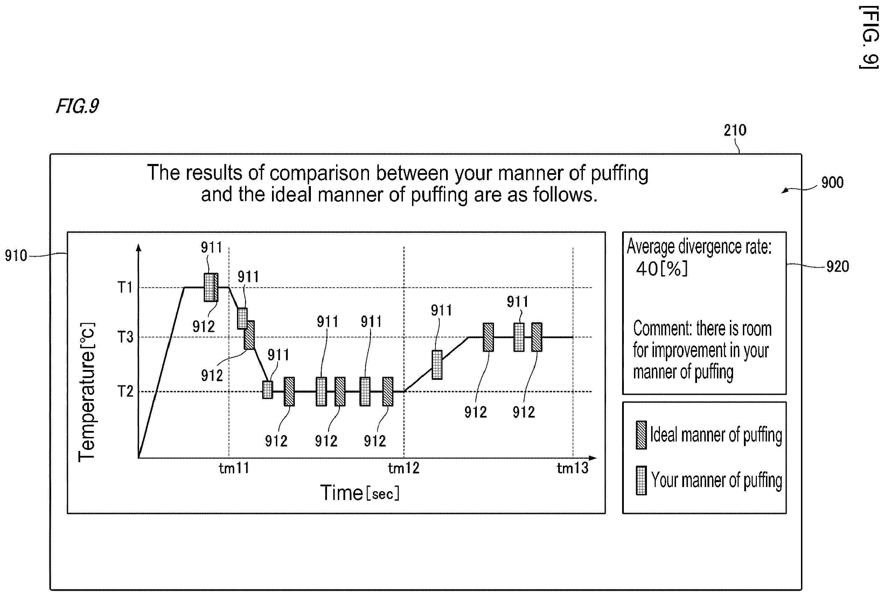

In the present embodiment, the comparison information is a visual comparison of the actual inhalation mode and the reference inhalation mode in order to allow the user to intuitively and easily grasp the degree of divergence of the actual inhalation mode from the reference inhalation mode. Note that a specific example of the comparison information will be described later using fig. 9.

[6. Example of Processes Performed by Control Unit of Terminal Device]

An example of the processes performed by the control unit 250 will be described. Fig. 6 is a flowchart showing an example of processes performed by the control unit 250.

As shown in fig. 6, initially, the control unit 250 performs a preference information acquisition process to obtain preference information representing a user's preferences pertaining to inhalation using the inhalation device 100 (step S1). A specific example of the preference information acquisition process will be described later using fig. 7.

The control unit 250 then performs a reference inhalation information acquisition process (step S2) to obtain reference inhalation information representing a reference inhalation mode that matches a user's preferences on the basis of the preference information acquired through the preference information acquisition process. A specific example of the reference inhalation information acquired by the reference inhalation information acquisition process and the reference inhalation information acquisition process will be described later using figs. 8A and 8B.

It should be noted that the preference information acquisition process and the reference inhalation information acquisition process may be performed only when the sequence of processes shown in fig. 6 is performed for the first time or when there is an execution request from the user. In other words, the preference information acquisition process and the reference inhalation information acquisition process need not be performed (i.e. may be skipped) on the second and subsequent executions of the sequence of processes shown in fig. 6. Then, if the preference information acquisition process and the reference inhalation information acquisition process are not performed, the processes of step S3 onwards described below may be performed in response to the terminal device 200 receiving the inhalation information from the inhalation device 100, for example.

The control unit 250 then performs an inhalation information acquisition process to acquire inhalation information (step S3). In the present embodiment, in the inhalation information acquisition process, the control unit 250 receives the inhalation information transmitted from the inhalation device 100 and obtains the received inhalation information.

The control unit 250 then performs a divergence calculation process of calculating an average divergence rate, which is an evaluation value of the degree of divergence of the actual inhalation mode from the reference inhalation mode, on the basis of the inhalation information acquired through the inhalation information acquisition process (step S4). A specific example of the divergence rate calculation process will be described later.

The control unit 250 then performs a comparison information generation process to generate comparison information comparing the actual inhalation mode and the reference inhalation mode (step S5). A specific example of the comparison information will be described later using fig. 9. The control unit 250 then causes the comparison information generated by the comparison information generation process to be displayed by the display unit 210 (step S6), to present the information to the user.

The control unit 250 then determines whether the average divergence rate calculated by the divergence rate calculation process is greater than or equal to a predetermined value (step S7). If it is determined that the average divergence rate is less than the predetermined value (step S7: No), i.e., if it determined that the degree of divergence of the actual inhalation mode from the reference inhalation mode is relatively small, the control 250 ends the process of the example shown in Fig. 6 as-is.

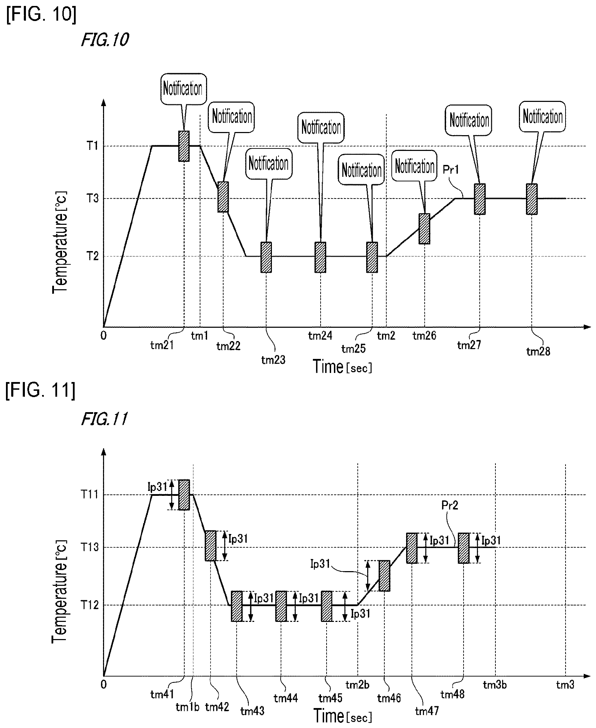

On the other hand, if it is determined that the average divergence rate is greater than or equal to the predetermined value (step S7: Yes), i.e., if it is determined that the divergence of the actual inhalation mode from the reference inhalation mode is somewhat large, the control unit 250 transmits the reference inhalation information acquired by the reference inhalation information acquisition process to the inhalation device 100 via the communication unit 240 (step S8), thereby causing the inhalation device 100 to perform inhalation assistance control for assisting inhalation in the reference inhalation mode representing the reference inhalation information, and ends the process of the example shown in fig. 6. A specific example of the inhalation assistance control will be described later using fig. 10.

<6-1. Preference Information Acquisition Process>

An example of the preference information acquisition process will be described next. The preference information acquisition process is, for example, a process of presenting a plurality of pre-prepared options to the user and acquiring preference information indicating the option selected by the user among the presented options.

Fig. 7 is a diagram showing an example of the preference information acquisition process. As shown in Fig. 7, in the preference information acquisition process, the control unit 250 causes the display unit 210 to display a message such as "Please indicate your preferred manner of puffing", together with an option Op1 stating "I want to puff slowly over a long time" and an option Op2 stating "I want to puff quickly over a short time", for example.

Then, as shown in fig. 7, when the option Op1 is selected (e.g., tapped) by the user, the control unit 250 acquires information indicating that the option Op1 has been selected as the preference information. On the other hand, if the option Op2 is selected by the user, the control unit 250 acquires information indicating that the option Op2 has been selected as the preference information.

<6-2. Reference Inhalation Information Acquisition Process and Reference Inhalation Information>

An example of the reference inhalation information acquisition process, and the reference inhalation information acquired by the reference inhalation information acquisition process will be described next. The reference inhalation information acquisition process is a process of transmitting, to the server 300, a delivery request including the preference information acquired by the preference information acquisition process, thereby acquiring, from the server 300, reference inhalation information representing the reference inhalation mode matching the preferences of the user (in other words, the reference inhalation mode corresponding to the option selected by the user).

Fig. 8A is a diagram showing an example of first reference inhalation information representing a first reference inhalation mode corresponding to option Op1. Fig. 8B is a diagram showing an example of second reference inhalation information representing a second reference inhalation mode corresponding to option Op2.

As described above, the reference inhalation mode is an inhalation mode in which the flavor to be tasted when the user inhales the aerosol generated by the inhalation device 100 is optimal, for example. More specifically, the reference inhalation mode includes, for example, a reference inhalation intensity as a reference value for the inhalation intensity, at which the flavor to be tasted when a user inhales the aerosol generated by the inhalation device 100 is optimal, and a reference inhalation time as a reference value for the inhalation time.

For example, in the first reference inhalation mode represented by the first reference inhalation information shown in fig. 8A, the times at which the elapsed time since the start of heating control is $tm21$[sec], $tm22$[sec], $tm23$[sec], $tm24$[sec], $tm25$[sec], $tm26$[sec], $tm27$[sec], and $tm28$[sec] are defined as reference inhalation times. Furthermore, in the first reference inhalation mode, the reference inhalation intensity corresponding to each of these reference inhalation times is defined as $Ip11$.

Thus, the first reference inhalation mode can be said to be the inhalation mode representing taking one puff having the inhalation intensity of $Ip11$ when the elapsed time since the start of heating control is $tm21$[sec], $tm22$[sec], $tm23$[sec], $tm24$[sec], $tm25$[sec], $tm26$[sec], $tm27$[sec], and $tm28$[sec]. In other words, in the inhalation mode represented by the first reference inhalation mode, the inhalation intensity of the nth puff (where n is a natural number from 1 to 8) is $Ip11$, and the inhalation time of the nth puff is when the elapsed time since the start of heating control is $tm2n$[sec].

It should be noted here that the reference inhalation intensities corresponding to each reference inhalation time of the first reference inhalation mode are all constant at $Ip11$, but is not limited to this, and may vary according to the reference inhalation time, for example.

On the other hand, in the second reference inhalation mode represented by the second reference inhalation information shown in fig. 8B, the times at which the elapsed time since the start of heating control is $tm31$[sec], $tm32$[sec], $tm33$[sec], $tm34$[sec], $tm35$[sec], $tm36$[sec], $tm37$[sec], and $tm38$[sec] are defined as reference inhalation times. For example, $tm31<tm21$, $tm32<tm22$, $tm33<tm23$, $tm34<tm24$, $tm35<tm25$, $tm36<tm26$, $tm37<tm27$, and $tm38<tm28$.

Furthermore, in the second reference inhalation mode, the reference inhalation intensity corresponding to each reference inhalation time is defined as $Ip21$. For example, here $Ip21>Ip11$.

Thus, the second reference inhalation mode can be said to be the inhalation mode representing taking one puff having the inhalation intensity of $Ip21$ when the elapsed time since the start of heating control is $tm31$[sec], $tm32$[sec], $tm33$[sec], $tm34$[sec], $tm35$[sec], $tm36$[sec], $tm37$[sec], and $tm38$[sec]. In other words, in the inhalation mode represented by the second reference inhalation mode, the inhalation intensity of the nth puff (where n is a natural number from 2 to 8) is $Ip21$, and the inhalation time of the nth puff is when the elapsed time since the start of heating control is $tm3n$[sec].

It should be noted here that the reference inhalation intensity corresponding to each reference inhalation time of the second reference inhalation mode is all constant at $Ip21$, but is not limited to this, and may vary according to the reference inhalation time, for example.

<6-3. Divergence Rate Calculation Process>

Next, an example of the divergence rate calculation process will be described. As described above, the divergence rate calculation process is a process of calculating the average divergence rate, which is an evaluation value of the degree of divergence of the actual inhalation mode from the reference inhalation mode. Here, the average divergence rate is the average value between the first divergence rate, which is an evaluation value of the degree of divergence of the actual inhalation intensity from the reference inhalation intensity, and the second divergence rate, which is the evaluation value of the degree of divergence of the actual inhalation time from the reference inhalation time.

The first divergence rate can be the average value of the inhalation intensity divergence rate for each puff taken during the heating control, for example. For example, if the inhalation intensity divergence rate of the nth puff taken during heating control is X(n), then X(n) can be expressed by equation (1) below.

$$X(n) = (IpAn - IpBn) / IpBn \times 100[\%]... (1)$$

In equation (1) above, IpAn is the inhalation intensity of the nth puff (or the average value of the inhalation intensity of the nth puff) taken during the heating control. Furthermore, IpBn is the reference inhalation intensity of the nth puff defined in the reference inhalation mode.

As an example, the inhalation information shown in (b) in fig. 4 is obtained by the inhalation information acquisition process, and the first reference inhalation information shown in Fig. 8A is obtained by the reference inhalation information acquisition process. In this case, the inhalation intensity divergence rate X(n = 1) of the first puff taken during the heating control is X(n = 1) = (Ip1 - Ip11) / Ip11 $\times$ 100. Similarly, the inhalation intensity divergence rate X(n = 2) of the second puff, the inhalation intensity divergence rate X(n = 3) of the third puff, ..., and the inhalation intensity divergence rate X(n = 8) of the eighth puff can also be obtained. In this case, the control unit 250 calculates (X(n = 1) + X(n = 2) + ... + X(n = 8)) / 8 as the first divergence rate.

Additionally, the second divergence rate can be, for example, the average value of the inhalation time divergence rate for each puff taken during the heating control. For example, if the inhalation time divergence rate of the nth puff taken during heating control is Y(n), then Y(n) can be expressed by equation (2) below.

$$Y(n) = (tmCn - tmDn) / tmDn \times 100[\%]... (2)$$

In equation (2) above, tmCn is the inhalation time of the nth puff (or the average value of the inhalation time of the nth puff) taken during the heating control. Furthermore, tmDn is the reference inhalation time of the nth puff defined in the reference inhalation mode.

As an example, the inhalation information shown in (b) in fig. 4 is obtained by the inhalation information acquisition process, and the first reference inhalation information shown in fig. 8A is obtained by the reference inhalation information acquisition process. In this case, the inhalation time divergence rate Y(n = 1) of the first puff taken during the heating control is Y(n = 1) = (tm11-tm21) / tm21 $\times$ 100. Similarly, the inhalation time divergence rate Y(n = 2) of the second puff, the inhalation time divergence rate Y(n = 3) of the third puff, ..., and the inhalation time divergence rate Y(n = 8) of the eighth puff can also be obtained. In this case, the control unit 250 calculates (Y(n = 1) + Y(n = 2) + ... + Y(n = 8)) / 8 as the second divergence rate.

In this way, when the control unit 250 calculates the first divergence rate and second divergence rate, the calculated average values of these, namely, (first divergence rate + second divergence rate) / 2 is further obtained as the average divergence rate.

<6-4. Comparison Information Generation Process and Comparison Information>

Next, an example of the comparison information generation process, and the comparison information generated by the comparison information generation process will be described. In the comparison information generation process, for example, the control unit 250 generates the comparison information on the basis of the reference inhalation information obtained by the reference inhalation information acquisition process, the inhalation information acquired by the inhalation information acquisition process, and the average divergence rate calculated by the divergence rate calculation process. The comparison information generated by this process is presented to the user by being displayed on the display unit 210 by the process of step S6, for example.

Fig. 9 is a diagram showing one example of the comparison information. As shown in fig. 9, the comparison information 900 includes a comparison image 910 and divergence rate information 920, for example.

The comparison image 910 is a visual comparison of the actual inhalation mode and the reference inhalation mode, and is an image obtained by superimposing a first image 911 representing the actual inhalation mode and a second image 912 representing the reference inhalation mode, for example. Here, the first image 911 can be an image configured by arranging an image (e.g. a rectangular image) having a vertical length that is based on the inhalation intensity of the puff at a position corresponding to the inhalation time of each puff taken during the heating control, for example. Furthermore, the second image 912 can be an image configured by arranging an image (e.g., a rectangular image) having a vertical length that is based on the reference inhalation intensity at a position corresponding to each reference inhalation time, for example. Such a comparison image 910 allows the degree of divergence of the actual inhalation mode from the reference inhalation mode to be intuitively indicated to the user in an easily understandable manner.

Furthermore, as shown in fig. 9, the divergence rate information 920 is information representing the calculated average divergence rate and comments that are based on the average divergence rate, for example. Comments that are based on the average divergence rate include, for example, "there is room for improvement in your manner of puffing" when the average divergence rate is 20[%] or more, and "your manner of puffing is close to ideal" when the average divergence rate is less than 20[%].

**In** the example shown in fig. 9, the calculated average divergence rate was 40[%], and thus the divergence rate information 920 is "Average divergence rate: 40[%] Comment: There is room for improvement in your manner of puffing". Such divergence rate information 920 allows the degree of divergence of the actual inhalation mode from the reference inhalation mode to be accurately indicated to the user in an easily understandable manner.

<6-5. Process of Executing Inhalation Assistance Control>

An example of inhalation assistance control will be described next. As previously described, when the average divergence rate is greater than or equal to a predetermined value, the control unit 250 transmits the reference inhalation information obtained through the reference inhalation information acquisition process to the inhalation device 100 via the communication unit 240, thereby causing the inhalation device 100 to perform inhalation assistance control to assist inhalation in the reference inhalation mode represented by the reference inhalation information. Here, the inhalation assistance control is, for example, control that causes the user to be notified that the reference inhalation time has been reached by the notification unit 113 of the inhalation device 100.

Fig. 10 is a diagram showing an example of inhalation assistance control. The example shown in fig. 10 is an example where the control unit 250 transmits the first reference inhalation information to the inhalation device 100, thereby instructing the inhalation device 100 to perform inhalation assistance control to assist inhalation in the first reference inhalation mode.

In the present example, the inhalation device 100 performs predetermined notification to the user by the notification unit 113 when the times at which the elapsed time since the start of heating control is tm21[sec], tm22 [sec], tm23[sec], tm24[sec], tm25[sec], tm26[sec], tm27[sec], and tm28[sec], or namely, when the reference inhalation time in the first reference inhalation mode has been reached. This notification should be able to indicate to the user that the reference inhalation time has been reached, and can, for example, be a vibration by a vibrating device included in the notification unit 113, light emission from a light emitting device included in the notification unit 113, etc. The notification may also cause a display device comprised in the notification unit 113 to display a predetermined image or output a predetermined sound from a sound output device comprised in the notification unit 113.

Such inhalation assistance control can notify the user that a reference inhalation time has been reached and can assist the user in taking an inhalation at the reference inhalation time. That is, it allows the user to make an inhalation at an appropriate time in terms of flavor, etc. of the aerosol generated by the inhalation device 100, and makes it possible to provide a better quality smoking experience for the user.

The inhalation device 100 may also be configured in the inhalation assistance control, for example the intensity of the vibration by the vibrating device of the notification unit 113; the light-emitting color of the light-emitting device, etc. may provide a notification such that the reference inhalation intensity in the reference inhalation mode is also indicative of to the user. This allows the user to draw with an appropriate inhalation intensity, such as in terms of flavor of the aerosol generated by the inhalation device 100, and makes it possible to provide a better quality smoking experience for the user.

As described above, the control unit 250 obtains inhalation information representing an actual inhalation mode taken on the inhalation device 100 and generates, on the basis of the acquired inhalation information, comparison information comparing the actual inhalation mode and the prescribed reference inhalation mode; present the generated comparison information to the user. This makes it possible for the comparison information to indicate, to the user, the degree of divergence of the actual inhalation mode from the reference inhalation mode, thereby enabling a new enjoyment to be provided to the user, such as seeking an inhalation mode closer to the reference inhalation mode with reference to this degree of divergence. Thus, it is possible to provide a more pleasurable and high-quality experience to the user and improve the merchantability of the inhalation device 100.

Furthermore, the reference inhalation mode is an inhalation mode in which the flavor to be tasted when the user inhales the aerosol generated by the inhalation device 100 is optimal, for example. This allows a higher-quality smoking experience to be provided to a user who performed inhalation in an inhalation mode close to the reference inhalation mode.

Furthermore, the comparison information generated by the control unit 250 includes an image representing the actual inhalation mode (e.g., the first image 911 shown in fig. 9) and an image representing the reference inhalation mode (e.g., the second image 912 shown in fig. 9). This allows the degree of divergence of the actual inhalation mode from the reference inhalation mode to be intuitively and easily grasped by the user.

The comparison information generated by the control unit 250 includes information (e.g., the divergence rate information 920 shown in fig. 9) representing the average divergence rate, which is an evaluation value of the degree of divergence of the actual inhalation mode from the reference inhalation mode. This allows the user to accurately grasp the degree of divergence of the actual inhalation mode from the reference inhalation mode.

The average divergence rate is calculated on the basis of the divergence of the actual inhalation intensity to the reference inhalation intensity included in the reference inhalation mode (e.g., the first divergence rate described above). This makes it possible to present the average divergence rate to the user, which is an evaluation value that takes into account the divergence of the actual inhalation intensity from the reference inhalation intensity.

The average divergence rate is calculated on the basis of the divergence of the actual inhalation time from the reference inhalation time included in the reference inhalation mode (e.g., the second divergence rate described above). This makes it possible to present the average divergence rate to the user, which is an evaluation value that takes into account the divergence of the actual inhalation time from the reference inhalation time.

Note that, in the foregoing, information representing the average divergence rate calculated from the first divergence rate and second divergence rate is provided to the user, but not limited to this. For example, information representing the first divergence rate or second divergence rate may be presented to the user instead of or in addition to information representing the average divergence rate.

The control unit 250 transmits the reference inhalation information representing the reference inhalation mode to the inhalation device 100 when the average divergence rate is greater than or equal to a predetermined value, and further performs a process of causing the inhalation device 100 to perform inhalation assistance control to assist with inhalation in the reference inhalation mode. This causes the inhalation device 100 to perform inhalation assistance control when there is a large degree of divergence of the actual inhalation mode from the reference inhalation mode, thereby making it possible to assist the user with inhalation in the reference inhalation mode. Thus, it is possible to provide a high-quality smoking experience to the user.

The inhalation assistance control can be control that, when the reference inhalation time has been reached, causes the user to be notified that the reference inhalation time has been reached by the notification unit 113 of the inhalation device 110, for example. This allows the user to be notified that the reference inhalation time has been reached, thereby making it possible to assist the user with performing inhalation in the reference inhalation time.

Furthermore, the reference inhalation mode may be different depending on the input result of the user. In this way, it is possible to suggest to the user the degree of divergence of the actual inhalation mode from the reference inhalation mode reflecting the user's preferences.

Additionally, in the inhalation system 10, the inhalation device 100 transmits inhalation information to the terminal device 200 at a predetermined time. The terminal device 200 comprises a display unit 210 capable of displaying information, generates comparison information on the basis of the inhalation information received from the inhalation device 100, and presents the generated comparison information to the user by causing the display unit 210 to display the comparison information. This makes it possible to indicate to the user the degree of divergence of the actual inhalation mode from the reference inhalation mode by causing the display unit 210 of the terminal device 200 to display the comparison information, even if the inhalation device 100 does not comprise a display unit such as the display unit 210. As such, the configuration of the inhalation device 100 can be simplified and the inhalation device 100 can be made smaller and cheaper than if a display unit such as the display unit 210 is provided to the inhalation device 100.

It should be noted that in the above, the heating control that is based on the heating profile Pr1 is configured to be performed, regardless of the results of input by the user (e.g., the aforementioned preference information), but is not limited to this. For example, the heating unit 121 of the inhalation device 100 may be configured to heat the aerosol source in a heating mode that is based on the results of input by the user.

More specifically, the control unit 250 may be configured to obtain, from the server 300, a heating profile matching the user's preferences and reference inhalation information representing the reference inhalation mode corresponding to the heating profile on the basis of the preference information acquired by the preference information acquisition process.

For example, when the option Op1 is selected by the user in the preference information acquisition process, the control unit 250 may transmit, to the server 300, a delivery request including preference information indicating that the option Op1 has been selected, thereby acquiring, from the server 300, the heating profile Pr1 which is a heating mode corresponding to the option Op1, and reference inhalation information representing the reference inhalation mode corresponding to the heating profile Pr1 (e.g., the first reference inhalation information shown in Fig. 8A). In this case, the control unit 250 may transmit the heating profile Pr1 obtained from the server 300 to the inhalation device 100 to thereby cause the control unit 116 of the inhalation device 100 to perform heating control that is based on the heating profile Pr1.

On the other hand, when the option Op2 is selected by the user in the preference information acquisition process, the control unit 250 may transmit, to the server 300, a delivery request including preference information indicating that the option Op2 has been selected, thereby acquiring, from the server 300, a heating profile Pr2 which is a heating mode corresponding to the option Op2 and which differs from the heating profile Pr1, and reference inhalation information representing the reference inhalation mode corresponding to the heating profile Pr2. In this case, the control unit 250 may transmit the heating profile Pr2 obtained from the server 300 to the inhalation device 100 to thereby cause the control unit 116 of the inhalation device 100 to perform heating control that is based on the heating profile Pr2.

Fig. 11 is a diagram showing an example of the heating profile Pr2 and the reference inhalation mode corresponding to the heating profile Pr2. In the heating profile Pr2, the target temperature when the elapsed time since the start of heating control is greater than or equal to 0[sec] but less than tm1b[sec] (where tmb1>0. For example, tm1b<tm1) is T11[°C] (e.g., T11>T1). Furthermore, the target temperature when the elapsed time since the start of heating control is greater than or equal to tm1b[sec] but less than tm2[sec] (where tm2>tm1b. For example, tm2b<tm2) is T12[°C] (where T2<T1. For example, T12>T2). Additionally, the target temperature when the elapsed time since the start of heating control is tm2b[sec] to tm3b[sec] (where tm3b>tm2b. For example, tm3b<tm3) is T13[°C] (where T12<T13<T11. For example, T1 3>T3).

Thus, according to the heating profile Pr2, as shown in fig. 11, the heating unit 121 (e.g., heating unit 121B) can be heated to T11[°C] in conjunction with the start of heating control, and then lowered in temperature to T12[°C], after which the heating unit can be heated again to T13[°C]. The heating control can then be ended when the elapsed time since the start of heating control is tm3b[s]. That is, according to the heating profile Pr2, the aerosol source (e.g., stick-type substrate 150) can be heated in a different mode than the heating profile Pr1.

Furthermore, as shown in fig. 11, in the reference inhalation mode corresponding to the heating profile Pr2, the times at which the elapsed time since the start of heating control is tm41[sec], tm42[sec], tm43[sec], tm44[sec], tm45[sec], tm46[sec], tm47[sec], and tm48[sec] are defined as reference inhalation times. Additionally, in the reference inhalation mode corresponding to the heating profile Pr2, the reference inhalation intensity corresponding to each of these reference inhalation times is defined as Ip31.

As explained above, the control unit 250 may transmit, to the inhalation device 100, a heating profile that is based on the results of input by the user, to instruct the inhalation device 100 to perform heating control that is based on the heating profile. In response to this instruction, the inhalation device 100 may be configured to generate an aerosol by heating the aerosol source by a heating mode represented by the heating profile that is based on the results of input by the user. The reference inhalation mode may be different depending on the heating mode (i.e., the heating profile). This allows the generation of an aerosol to be performed by a heating mode that reflects the user's preferences and also makes it possible to indicate, to the user, the degree of divergence of the actual inhalation mode from the appropriate reference inhalation mode according to the heating mode.

Note that, in the foregoing, the control unit 250 is configured to present the comparison information to the user by causing the information to be displayed on the display unit 210 of the terminal device 200, but is not limited to this. For example, the control unit 250 transmits the comparison information to the inhalation device 100, thereby presenting the information to the user via the notification unit 113 of the inhalation device 100.

The above described examples of the information processing device of the present disclosure were realized by the terminal device 200 that is able to communicate with the inhalation device 100, but the information processing device is not limited to this. For example, the information processing device of the present disclosure may be realized by the inhalation device 100. In this case, for example, the control unit 116 of the inhalation device 100 may perform each process performed by the control unit 250 of the terminal device 200 described above, and present the comparison information to the user via the notification unit 113 of the inhalation device 100.

The information processing device of the present disclosure may also be realized, for example, by a server (e.g., server 300) able to communicate with the inhalation device 100 via a predetermined network such as the Internet. In this case, for example, the control unit (e.g., CPU) of the server performs each process performed by the control unit 250 of the terminal device 200 as described above, and transmits the process results to the inhalation device 100, thereby presenting the comparison information to the user via the notification unit 113 of the inhalation device 100.

Note that the information presentation method described in the present embodiment can be realized by executing a pre-prepared program (information presentation program) on a computer. The information presentation program is stored in a computer-readable storage medium, and is performed by being read out from the storage medium, for example. The information presentation program may also be provided in a form stored in a non-volatile (non-transitory) storage medium such as a flash memory, or may be provided over a network such as the Internet. Furthermore, in the present embodiment, the computer that performs this information presentation program was the terminal device 200 (e.g., a CPU constituting the control unit 250), but the present invention is not limited to this. For example, the computer that performs this information presentation program may be included in the inhalation device 100 (e.g., a CPU constituting the control unit 116) or in a server device that can communicate with the inhalation device 100 or the terminal device 200.

One embodiment of the information processing device, inhalation system, and the information presentation method of the present disclosure has been described above with reference to the drawings, but it goes without saying that the present invention is not limited to this embodiment. It is obvious that a person skilled in the art will be able to conceive of a number of variant examples or modified examples within the scope disclosed in the claims, and any such variant examples or modified examples are naturally understood to fall within the technical scope of the present disclosure. Furthermore, the components in the embodiment described above may be arbitrarily combined within a range not deviating from the spirit of the invention.

The present specification etc. sets forth at least the following features. Corresponding components etc. in the embodiment described above are shown by way of example in parentheses, but are not limited thereto.

(1) An information processing device (terminal device 200) comprising a control unit (control unit 250) that performs the processes of: acquiring inhalation information representing an actual inhalation mode used for an inhalation device (inhalation device 100, 100A, 100B) for delivering an aerosol which can be inhaled by a user (step S3);

generating, on the basis of the inhalation information, comparison information (comparison information 900) comparing the actual inhalation mode and a prescribed reference inhalation mode (step S5); and
presenting the comparison information to the user (step S6).

By means of (1), it is possible for the comparison information to indicate, to the user, the degree of divergence of the actual inhalation mode from the reference inhalation mode, thereby enabling a new enjoyment to be provided to the user, such as seeking an inhalation mode closer to the reference inhalation mode with reference to this degree of divergence. Thus, it is possible to provide a more pleasurable and high-quality experience to the user.

(2) The information processing device as disclosed in (1), wherein

the inhalation device generates the aerosol containing a flavor component, and
the reference inhalation mode is an inhalation mode in which the flavor to be tasted when the user inhales the aerosol generated by the inhalation device is optimal.

By means of (2), it is possible to provide a higher-quality smoking experience to a user who performed inhalation in an inhalation mode close to the reference inhalation mode.

(3) The information processing device as disclosed in (1) or (2), wherein the comparison information includes an image (first image 911) representing the actual inhalation mode and an image (second image 912) representing the reference inhalation mode.

By means of (3), the degree of divergence of the actual inhalation mode from the reference inhalation mode can be intuitively grasped by the user in an easily understandable manner.

(4) The information processing device as disclosed in any one of (1) to (3), wherein the comparison information includes information (divergence rate information 920) representing an evaluation value of the degree of divergence of the actual inhalation mode from the reference inhalation mode.

By means of (4), the degree of divergence of the actual inhalation mode from the reference inhalation mode can be accurately grasped by the user.

(5) The information processing device as disclosed in (4), wherein

the actual inhalation mode includes an inhalation intensity pertaining to an inhalation performed on the inhalation device,
the reference inhalation mode includes a reference inhalation intensity serving as a reference value at which the flavor to be tasted when the user inhales the aerosol generated by the inhalation device is optimal, and
the evaluation value is calculated on the basis of the divergence of the inhalation intensity from the reference inhalation intensity.

By means of (5), it is possible to present, to the user, an evaluation value which takes into account the divergence of the actual inhalation intensity from the reference inhalation intensity.

(6) The information processing device as disclosed in (4) or (5), wherein

the actual inhalation mode includes an inhalation time pertaining to an inhalation performed on the inhalation device,
the reference inhalation mode includes a reference inhalation time serving as a reference value at which the flavor to be tasted when the user inhales the aerosol generated by the inhalation device is optimal, and
the evaluation value is calculated on the basis of the divergence of the inhalation time from the reference inhalation time.

By means of (6), it is possible to present, to the user, an evaluation value which takes into account the divergence of the actual inhalation time from the reference inhalation time.

(7) The information processing device as disclosed in any one of (4) to (6), wherein the control unit further performs a process (step S8) of causing the inhalation device to perform inhalation assistance control for assisting with inhalation in the reference inhalation mode when the evaluation value is greater than or equal to a predetermined value.

By means of (7), when the degree of divergence of the actual inhalation mode from the reference inhalation mode is large, the inhalation assistance control can be performed to assist the user with inhalation in the reference inhalation mode.

(8) The information processing device as disclosed in (7), wherein

the inhalation device comprises a notification unit (notification unit 113, 113A, 113B) capable of providing a notification to the user, and
the inhalation assistance control is control for causing the notification unit to notify the user that the reference inhalation time has been reached when having reached the reference inhalation time, which serves as a reference value at which the flavor to be tasted when the user inhales the aerosol generated by the inhalation device is optimal.

By means of (8), it is possible for the user to be notified that the reference inhalation time has been reached, thereby making it possible to assist the user with performing inhalation in the reference inhalation time.

(9) The information processing device as disclosed in any one of (1) to (8), wherein the reference inhalation mode differs depending on a result of input by the user.

By means of (9), it is possible to indicate, to the user, the degree of divergence of the actual inhalation mode from the reference inhalation mode reflecting the user's preferences.

(10) The information processing device as disclosed in (9), wherein

the inhalation device generates the aerosol by heating an aerosol source with a heating unit (heating unit 121, 121B), the heating unit heats the aerosol source in a heating mode (heating profiles Prl, Pr2) that is based on a result of input by the user, and
the reference inhalation mode differs depending on the heating mode.

By means of (10), it is possible to indicate, to the user, the degree of divergence of the actual inhalation mode from an appropriate reference inhalation mode according to the heating mode of the heating unit.

(11) An inhalation system (inhalation system 10) including the information processing device as disclosed in any one of (1) to (10), and the inhalation device capable of communicating with the information processing device, wherein

the inhalation device transmits the inhalation information to the information processing device at a predetermined timing, and
the information processing device
is a terminal device that comprises a display unit (display unit 210) capable of displaying information, and that is used by the user,
generating the comparison information on the basis of the inhalation information received from the inhalation device, and
presenting the comparison information to the user by causing the display unit to display the comparison information.

By means of (11), it is possible to indicate, to the user, the degree of divergence of the actual inhalation mode from the reference inhalation mode by causing the display unit of the terminal device to display the comparison information, even if the inhalation device does not comprise a display unit. As such, the configuration of the inhalation device can be simplified and the inhalation device can be made smaller and cheaper than if a display unit is provided to the inhalation device.

(12) An information presentation method in which a computer (terminal device 200, control unit 250) performs the processes of:

acquiring inhalation information representing an actual inhalation mode used for an inhalation device (inhalation device 100, 100A, 100B) for delivering an aerosol which can be inhaled by a user (step S3);
generating, on the basis of the inhalation information, comparison information (comparison information 900) comparing the actual inhalation mode and a prescribed reference inhalation mode (step S5); and
presenting the comparison information to the user (step S6).

By means of (12), it is possible for the comparison information to indicate, to the user, the degree of divergence of the actual inhalation mode from the reference inhalation mode, thereby enabling a new enjoyment to be provided to the user, such as seeking an inhalation mode closer to the reference inhalation mode with reference to this degree of divergence. Thus, it is possible to provide a more pleasurable and high-quality experience to the user.

(13) An information presentation program for causing a computer (terminal device 200, control unit 250) to perform the processes of:

acquiring inhalation information representing an actual inhalation mode used for an inhalation device (inhalation device 100, 100A, 100B) for delivering an aerosol which can be inhaled by a user (step S3);

generating, on the basis of the inhalation information, comparison information (comparison information 900) comparing the actual inhalation mode and a prescribed reference inhalation mode (step S5); and

presenting the comparison information to the user (step S6).

By means of (13), it is possible for the comparison information to indicate, to the user, the degree of divergence of the actual inhalation mode from the reference inhalation mode, thereby enabling a new enjoyment to be provided to the user, such as seeking an inhalation mode closer to the reference inhalation mode with reference to this degree of divergence. Thus, it is possible to provide a more pleasurable and high-quality experience to the user.

(14) A computer-readable storage medium storing the information presentation program as disclosed in (13).

By means of (14), it is possible to cause a computer to perform the information presentation program as disclosed in (13).

[REFERENCE SIGNS LIST]

10 Inhalation system
100, 100A, 100B Inhalation device
200 Terminal device (information processing device)
250 Control unit
900 Comparison information

**Claims**

1.    An information processing device comprising a control unit that performs the processes of:

acquiring inhalation information representing an actual inhalation mode used for an inhalation device for delivering an aerosol which can be inhaled by a user;

generating, on the basis of the inhalation information, comparison information in which the actual inhalation mode and a prescribed reference inhalation mode are compared; and

presenting the comparison information to the user.

2.    The information processing device as claimed in claim 1, wherein

the inhalation device generates the aerosol containing a flavor component, and

the reference inhalation mode is an inhalation mode in which the flavor to be tasted when the user inhales the aerosol generated by the inhalation device is optimal.

3.    The information processing device as claimed in claim 1 or 2, wherein

the comparison information includes an image representing the actual inhalation mode and an image representing the reference inhalation mode.

4.    The information processing device as claimed in any one of claims 1 to 3, wherein

the comparison information includes information representing an evaluation value of the degree of divergence of the actual inhalation mode from the reference inhalation mode.

5.    The information processing device as claimed in claim 4, wherein

the actual inhalation mode includes an inhalation intensity pertaining to an inhalation performed on the inhalation device,
the reference inhalation mode includes a reference inhalation intensity serving as a reference value at which the flavor to be tasted when the user inhales the aerosol generated by the inhalation device is optimal, and
the evaluation value is calculated on the basis of the divergence of the inhalation intensity from the reference inhalation intensity.

6. The information processing device as claimed in claim 4 or 5, wherein

the actual inhalation mode includes an inhalation time pertaining to an inhalation performed on the inhalation device,
the reference inhalation mode includes a reference inhalation time serving as a reference value at which the flavor to be tasted when the user inhales the aerosol generated by the inhalation device is optimal, and
the evaluation value is calculated on the basis of the divergence of the inhalation time from the reference inhalation time.

7. The information processing device as claimed in any one of claims 4 to 6, wherein
the control unit further performs a process of causing the inhalation device to perform inhalation assistance control for assisting with inhalation in the reference inhalation mode when the evaluation value is greater than or equal to a predetermined value.

8. The information processing device as claimed in claim 7, wherein

the inhalation device comprises a notification unit capable of providing a notification to the user, and
the inhalation assistance control is control for causing the notification unit to notify the user that the reference inhalation time has been reached when having reached the reference inhalation time, which serves as a reference value at which the flavor to be tasted when the user inhales the aerosol generated by the inhalation device is optimal.

9. The information processing device as claimed in any one of claims 1 to 8, wherein
the reference inhalation mode differs depending on a result input by the user.

10. The information processing device as claimed in claim 9, wherein

the inhalation device generates the aerosol by heating an aerosol source with a heating unit,
the heating unit heats the aerosol source in a heating mode that is based on a result input by the user, and
the reference inhalation mode differs depending on the heating mode.

11. An inhalation system including the information processing device as claimed in any one of claims 1 to 10, and the inhalation device capable of communicating with the information processing device, wherein

the inhalation device transmits the inhalation information to the information processing device at a predetermined timing, and
the information processing device
is a terminal device that comprises a display unit capable of displaying information, and that is used by the user,
generating the comparison information on the basis of the inhalation information received from the inhalation device, and
presenting the comparison information to the user by causing the display unit to display the comparison information.

12. An information presentation method in which a computer performs the processes of:

acquiring inhalation information representing an actual inhalation mode used for an inhalation device for delivering an aerosol which can be inhaled by a user;
generating, on the basis of the inhalation information, comparison information in which the actual inhalation mode and a prescribed reference inhalation mode are compared; and
presenting the comparison information to the user.

[FIG. 1]

*FIG.1*

EP 4 613 138 A1

FIG.2A

100A

[FIG. 2B]

*FIG.2B*

[FIG. 3]

*FIG.3*

[FIG. 4]

EP 4 613 138 A1

*FIG.4*

(a)

(b)

| Inhalation information | | |
|---|---|---|
| Puff | Inhalation time | Inhalation intensity |
| First puff | tm11 | Ip1 |
| Second puff | tm12 | Ip2 |
| Third puff | tm13 | Ip3 |
| Fourth puff | tm14 | Ip4 |
| Fifth puff | tm15 | Ip5 |
| Sixth puff | tm16 | Ip6 |
| Seventh puff | tm17 | Ip7 |
| Eighth puff | tm18 | Ip8 |

[FIG. 5]

*FIG.5*

200

210
250

Display unit ↔

220

Input unit ↔

230

Control unit

Memory unit ↔

240

Communication unit ↔

[FIG. 6]

*FIG.6*

START

Acquire preference information ⟶ S1

Acquire reference inhalation information ⟶ S2

Acquire inhalation information ⟶ S3

Calculate average divergence rate ⟶ S4

Generate comparison information ⟶ S5

Display comparison information ⟶ S6

Is average divergence rate greater than or equal to predetermined value? — S7 — No

Yes — S8

Transmit reference inhalation information

END

24

[FIG. 7]

*FIG.7*

210

Please indicate your
preferred manner of puffing

I want to puff
slowly over a
long time

Op1

I want to puff
quickly over a
short time

Op2

[FIG. 8A]

*FIG.8A*

[FIG. 8B]

*FIG.8B*

[FIG. 9]

EP 4 613 138 A1

FIG.9

The results of comparison between your manner of puffing
and the ideal manner of puffing are as follows.

Average divergence rate:
40[%]

Comment: there is room
for improvement in your
manner of puffing

▨ Ideal manner of puffing

▨ Your manner of puffing

[FIG. 10]

*FIG.10*

[FIG. 11]

*FIG.11*

## INTERNATIONAL SEARCH REPORT

| International application No. |
|---|
| **PCT/JP2022/041181** |

| | |
|---|---|
| **A.** | **CLASSIFICATION OF SUBJECT MATTER** |

*A24F 40/65*(2020.01)i; *A24F 40/53*(2020.01)i; *A24F 40/57*(2020.01)i
FI:   A24F40/65; A24F40/53; A24F40/57

According to International Patent Classification (IPC) or to both national classification and IPC

| | |
|---|---|
| **B.** | **FIELDS SEARCHED** |

Minimum documentation searched (classification system followed by classification symbols)

A24F40/00-47/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| X | JP 2021-517819 A (NICOVENTURES TRADING LTD.) 29 July 2021 (2021-07-29) paragraphs [0004], [0005], [0023]-[0026], [0037]-[0046], [0056], [0079]-[0092], fig. 6-8 | 1-12 |
| A | JP 2022-543353 A (KT & G CORP.) 12 October 2022 (2022-10-12) entire text, all drawings | 1-12 |
| A | CN 111466621 A (SHENZHEN WOODY VAPES TECHNOLOGY CO., LTD.) 31 July 2020 (2020-07-31) entire text, all drawings | 1-12 |
| A | JP 2022-538120 A (PNEUMA RESPIRATORY, INC.) 31 August 2022 (2022-08-31) entire text, all drawings | 1-12 |

☐ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **09 December 2022** | **27 December 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2022/041181**

| Patent document cited in search report | | | Publication date (day/month/year) | Patent family member(s) | | | Publication date (day/month/year) |
|---|---|---|---|---|---|---|---|
| JP | 2021-517819 | A | 29 July 2021 | US 2021/0259316 A1 paragraphs [0005], [0006], [0031]-[0034], [0045]-[0054], [0062], [0084]-[0097], fig. 6-8 WO 2019/207312 A1 EP 3784077 A1 KR 10-2020-0129158 A | | | |
| JP | 2022-543353 | A | 12 October 2022 | US 2022/0264956 A1 entire text, all drawings WO 2022/010261 A1 CN 114521113 A KR 10-2022-0006406 A | | | |
| CN | 111466621 | A | 31 July 2020 | (Family: none) | | | |
| JP | 2022-538120 | A | 31 August 2022 | US 2022/0296823 A1 entire text, all drawings WO 2020/264501 A1 EP 3990071 A1 CN 114206421 A | | | |

Form PCT/ISA/210 (patent family annex) (January 2015)

**REFERENCES CITED IN THE DESCRIPTION**

**Patent documents cited in the description**

- JP 2015524260 A

- WO 2015161402 A1